(19) **Europäisches Patentamt / European Patent Office / Office européen des brevets**

(11) **EP 1 784 232 B1**

(12) **EUROPEAN PATENT SPECIFICATION**

(45) Date of publication and mention
of the grant of the patent:
**14.10.2009  Bulletin 2009/42**

(51) Int Cl.:
*A61L 15/44* (2006.01)      *A61L 15/58* (2006.01)
*A61L 15/48* (2006.01)

(21) Application number: **05784255.1**

(22) Date of filing: **11.08.2005**

(86) International application number:
**PCT/US2005/028417**

(87) International publication number:
**WO 2006/020708 (23.02.2006 Gazette 2006/08)**

(54) **BIOLOGICALLY-ACTIVE ADHESIVE ARTICLES AND METHODS OF MANUFACTURE**

BIOLOGISCH WIRKSAME KLEBEARTIKEL UND HERSTELLUNGSVERFAHREN

ARTICLES ADHESIFS BIOLOGIQUEMENT ACTIFS ET PROCEDES DE PRODUCTION

(84) Designated Contracting States:
**AT BE BG CH CY CZ DE DK EE ES FI FR GB GR HU IE IS IT LI LT LU LV MC NL PL PT RO SE SI SK TR**

(30) Priority: **12.08.2004  US 917102**

(43) Date of publication of application:
**16.05.2007  Bulletin 2007/20**

(73) Proprietor: **3M Innovative Properties Company**
**St. Paul MN 55133-3427 (US)**

(72) Inventors:
 • **YLITALO, Caroline M.**
  **Saint Paul,**
  **Minnesota 55133-3427 (US)**
 • **TOKIE, Jeffrey H.**
  **Saint Paul,**
  **Minnesota 55133-3427 (US)**
 • **SCHOLZ, Matthew T.**
  **Saint Paul,**
  **Minnesota 55133-3427 (US)**
 • **RAO, Prabhakara S.**
  **Saint Paul,**
  **Minnesota 55133-3427 (US)**

 • **KRAMPE, Stephen E.**
  **Saint Paul,**
  **Minnesota 55133-3427 (US)**
 • **HENDRICKSON, Mark J.**
  **Saint Paul,**
  **Minnesota 55133-3427 (US)**
 • **ELLIOTT, Peter T.**
  **Saint Paul,**
  **Minnesota 55133-3427 (US)**
 • **BURTON, Scott A.**
  **Saint Paul,**
  **Minnesota 55133-3427 (US)**

(74) Representative: **von Kreisler Selting Werner**
**Patentanwälte**
**Deichmannhaus am Dom**
**Bahnhofsvorplatz 1**
**50667 Köln (DE)**

(56) References cited:
**EP-A- 0 524 776          US-A- 4 310 509**
**US-A1- 2002 001 608     US-A1- 2003 054 025**
**US-A1- 2003 175 333**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

BACKGROUND OF THE INVENTION

Wound care articles, such as bandages and wound dressings, are available in a variety of designs to protect wounds from environmental conditions during the healing process. Pressure sensitive adhesive (PSA) layers are commonly used to adhere the wound care articles to the skin of patients. Typically the PSA layers are coated onto backing substrates, where the backing substrates may be a variety of materials, such as flexible films, foam, woven materials, non-woven materials, or gauze.

In general, wounds generally heal more effectively in moist environments. However, such environments also increase the risk of bacterial infection. To reduce this risk, many wound care articles are designed to release biological actives, such as antimicrobials, to prevent or treat bacterial infections. As such, the use of biological actives with PSA layers allows the wound care articles to retain contact with a wound site, and also release the biological actives to the skin to reduce the risk of infections.

Document US 2003/0054025 describes a method of coating an adhesive layer on a pressure adhesive sensitive article, the method comprising: inkjet non-contact printing deposition of a fluid solution onto the adhesive layer, the fluid solution comprising a biological active (containing silver salt and ammonium). Document US 2003/0175333 describes a method of coating an adhesive layer on a pressure adhesive sensitive article, the method comprising: spray deposition of a fluid solution onto the adhesive layer, the fluid solution comprising a biological active (containing silver salt and ammonium). These prior art documents mentioned the diffusion of the biological active in the adhesive.

However, biological actives applied to the wound care articles typically affect the physical properties of the PSA layers. For example, coating biological actives on PSA layers may act as plasticizers that reduce the adhesive strength of the PSA layers. This accordingly reduces the effectiveness of the wound care article. As such, there is a need for adhesive articles prepared with biological actives, where the adhesive layers retain good physical properties.

BRIEF SUMMARY OF THE INVENTION

The present invention relates to a method of coating an adhesive layer, the method including non-contact depositing a fluid solution onto the adhesive layer, where the fluid solution comprising a biological active, and where the fluid solution exhibits a Hildebrand solubility parameter of at least 3.7 MegaPascals$^{1/2}$ greater than a Hildebrand solubility parameter of the adhesive layer. The fluid solution is then allowed to substantially dry.

The present invention further relates to a method of coating an adhesive layer, the method including combining a silver-containing compound, an ammonium-containing compound, and an aqueous solvent, thereby forming a fluid solution that exhibits a Hildebrand solubility parameter of at least 3.7 MegaPascals$^{1/2}$ greater than a Hildebrand solubility parameter of the adhesive layer. The fluid solution is non-contact deposited onto the adhesive layer and allowed to substantially dry.

The present invention further relates to an article comprising an adhesive layer and a biological active deposited on the adhesive layer by non-contact deposition of a fluid solution comprising the biological active. The fluid solution exhibits a Hildebrand solubility parameter of at least 3.7 MegaPascals$^{1/2}$ greater than a Hildebrand solubility parameter of the adhesive layer.

BRIEF DESCRIPTION OF THE DRAWINGS

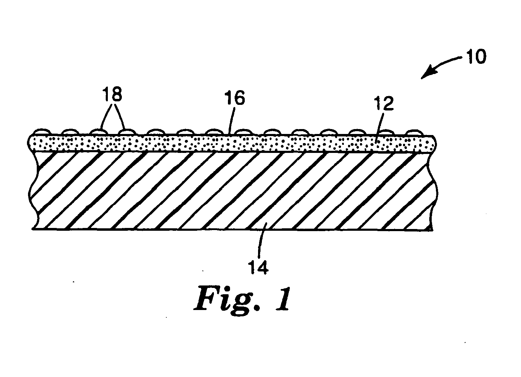

FIG. 1 is a sectional view of a wound dressing article of the present invention.

While FIG. 1 sets forth only one embodiment of the invention, other embodiments are also contemplated, as noted in the discussion. The figure may not be drawn to scale.

DETAILED DESCRIPTION

The present invention relates to a method of applying a biological active to an adhesive article (depicted sectionally in FIG. 1 as an article 10) by non-contact deposition. As shown in FIG. 1, the article 10 includes an adhesive layer 12 disposed on a backing substrate 14, where the adhesive layer includes a surface 16. The method of the present invention involves providing a fluid solution 18 that contains one or more biological actives, and which exhibits low solubility with the adhesive layer 12. The fluid solution 18 is applied to the surface 16 of the adhesive layer 12 by non-contact deposition, and is allowed to substantially dry. Because the fluid solution 18 exhibits low solubility with the adhesive layer 12, the fluid solution 18 does not significantly diffuse into the bulk of the adhesive layer 12. As such, the biological active remains concentrated on or near the surface 16 of the adhesive layer 12 when the fluid solution 18 substantially dries.

Because the biological active remains concentrated on or near the surface 16, the article 10 may incorporate low concentrations of the biological active, while still exhibiting effective levels of antimicrobial activity, as measured by the Zone of Inhibition Test described below. Effective antimicrobial activity includes primary zones of inhibition on the coated article of 8 mm, more preferably 10 mm, and even more preferably 12 mm.

The low concentrations of the biological active reduces interactions between the biological active and the adhesive layer 12. This allows the adhesive layer 12 to retain desirable physical properties (e.g., good adhesive strengths, long wear, high moisture vapor transmission, preferred modulus values, transparency and absorbency) despite the presence of the biological active. This is particularly useful where the article 10 is a PSA wound dressing article. The article 10 retains good adherence to the skin of a patient during use, and releases the biological active to the wound site to reduce the risk of infections.

The fluid solution 18 of the present invention may include a variety of different biological actives, such as antimicrobials, antibiotics, antifungals, antivirals, and antiseptics (discussed in further detail below). Because the biological active remains concentrated on or near the surface 16, the biological active is not required to diffuse through the bulk of the adhesive layer 12 and the backing substrate 14 before being released. As such, when the article 10 is applied to a wound site, the biological active may rapidly release from the adhesive layer 12 to protect against infections.

A suitable manner for measuring the solubility of the fluid solution 18 and the adhesive layer 12 is with Hildebrand solubility parameters ($\delta$). The Hildebrand solubility parameter, as used herein, represents the square root of the cohesive energy density of a material, and is represented by the following equation:

$$\delta = \sqrt{\frac{(\Delta H - RT)}{V}}$$

where $\Delta H$ is the molar vaporization enthalpy of the material, R is the universal gas constant, T is the absolute temperature, and V is the molar volume of the solution. Hildebrand solubility parameters are generally provided in conventional units of (calories/centimeter$^3$)$^{1/2}$ ((cal/cm$^3$)$^{1/2}$) and in SI units of megaPascals$^{1/2}$ (MPa$^{1/2}$).

The level of solubility between substances (e.g., between the fluid solution and the adhesive layer) is based on the difference in the Hildebrand solubility parameters of the substances. Generally, the closer the Hildebrand solubility parameters between two substances are, the more soluble and compatible they are. Conversely, the further apart the Hildebrand solubility parameters of the two substances are, the less soluble the two substances are.

Methods to calculate the solubility parameter based on group contribution effects using the structure to estimate the cohesive energy density are described in D.W. Van Krevelen's Properties of Polymers. Elsevier Science, 3rd Ed. November 1, 1997; and D.H. Kaeble's Computer-Aided Design of Polymer and Composites. Marcel Dekker, Inc. 1985. Examples of Hildebrand solubility parameters for various solvents and polymers are tabulated in Barton, A. F. M., Handbook of Solubility and Other Cohesion Parameters, 2nd Ed., CRC Press, Boca Raton, Fla. (1991); Barton, A, F. M., Handbook of Polymer-Liquid Interaction Parameters and Solubility Parameters, CRC Press, Boca Raton, Fla. (1990); Polymer Handbook, 3rd Ed., J. Brandrup & E. H. Immergut, Eds. John Wiley, NY, pp 519-557 (1989); and Applied Polymer Science, Eds. J. Kenneth Carver & Roy M. Tess, Organic Coatings and Plastics Chemistry, ACS Publication 1975 (Library of Congress Catalog Control No: 75-23010); Table I provides examples of Hildebrand solubility parameters for various solvents and acrylic-based adhesive polymers.

TABLE 1

| Component | Hildebrand Solubility Parameter (cal/cm$^3$)$^{1/2}$ | Hildebrand Solubility Parameter (MPa)$^{1/2}$ |
|---|---|---|
| Water | 23.4 | 47.9 |
| Glycerol | 21.1 | 43.2 |
| Ethylene glycol | 16.3 | 33.3 |
| Propylene glycol | 14.8 | 30.3 |
| Methanol | 14.5 | 29.7 |
| Ethanol | 12.7 | 26.0 |
| Isopropanol | 11.5 | 23.5 |

(continued)

| Component | Hildebrand Solubility Parameter $(cal/cm^3)^{1/2}$ | Hildebrand Solubility Parameter $(MPa)^{1/2}$ |
| --- | --- | --- |
| Acrylic acid | 12.0 | 24.5 |
| Poly(methyl acrylate) | 9.7 | 19.8 |
| Poly(methyl methacrylate) | 9.4 | 19.2 |
| Tetrahydrofurfurylacrylate | 9.3 | 19.0 |
| Poly(ethyl acrylate) | 9.2 | 18.8 |
| Isobornylacrylate | 9.2 | 18.8 |
| Poly(ethyl methacrylate) | 9.0 | 18.4 |
| Ethoxy-ethoxyethylacrylate | 9.0 | 18.4 |
| Poly(n-Butyl acrylate) | 8.7 | 17.8 |
| Poly(n-Butyl methacrylate) | 8.7 | 17.8 |
| Hexadiene diacrylate | 7.9 | 16.2 |
| Isooctylacrylate | 7.8 | 16.0 |
| Polydimethylsiloxane | 7.6 | 15.5 |
| Tetrachlorosilane | 7.4 | 15.1 |

As shown in Table 1, the Hildebrand solubility parameters of the provided solvents are substantially different from typical acrylic-based adhesive polymers. As such, there is little interaction between them, especially with solvents such as water.

As used herein, the Hildebrand solubility parameter of a mixture of multiple substances is based on the weighted average of the Hildebrand solubility parameters of the individual substances, based on the total weight of the mixture. Examples of suitable solubilities for the fluid solution of the present invention include Hildebrand solubility parameters of at least 3.7 $MPa^{1/2}$ (1.8 $(cal/cm^3)^{1/2}$) greater than the Hildebrand solubility parameter of the adhesive layer 12 being coated. Particularly suitable solubilities for the fluid solution of the present invention include Hildebrand solubility parameters of at least 8.0$MPa^{1/2}$ (3.9 $(cal/cm^3)^{1/2}$) greater than the Hildebrand solubility parameter of the adhesive layer being coated. Even more particularly suitable solubilities for the fluid solution of the present invention include Hildebrand solubility parameters of at least 15.0. $MPa^{1/2}$ (7.3 $(cal/cm^3)^{1/2}$) greater than the Hildebrand solubility parameter of the adhesive layer being coated. Such differences in Hildebrand solubility parameters provide low solubilities between the fluid solution of the present invention and the adhesive layer being coated.

Another suitable manner for measuring the solubility of the fluid solution 18 and the adhesive layer 12 is with critical surface tensions, which are related to the thermodynamic surface free energies of the substances. In general, the critical surface tension of a substance is directly proportional to the Hildebrand solubility parameter of the substance. As such, substances of similar critical surface tensions are soluble and interact and substances with differing critical surface tensions are not soluble and do not interact. However, anomalies of dispersion, polar, and hydrogen-bonding interactions are characterized differently with the critical surface tension and the Hildebrand solubility parameter. Examples of critical surface tensions for various solvents and polymers, and the correlating Hildebrand solubility parameters, are tabulated in Ed. I. Skeist & V. N. Reinhold, Handbook of Adhesives, 3rd Ed. (1990), which is incorporated by reference in its entirety.

Non-contact deposition techniques suitable for the present invention are generally independent of the surface being coated (e.g., the surface 16 of the adhesive layer 12). As such, a non-contact deposition mechanism may be moved in a transverse direction to the surface 16 being coated, while imparting substantially no transverse force to the surface 16. In contrast to contact coating techniques, non-contact deposition allows the same processing equipment to be used for coating a variety of different surfaces 16 without requiring changes in formulations or process parameters. Examples of suitable non-contact deposition techniques include inkjet printing, spray atomization deposition, electrostatic deposition, microdispensing, and mesoscale deposition. Particularly suitable non-contact deposition techniques include inkjet printing and spray atomization deposition.

Inkjet printing operates by ejecting the fluid solution 18 onto the adhesive layer 12 in controlled patterns of fluid droplets. Examples of suitable inkjet printing methods include thermal inkjet, continuous inkjet, piezo inkjet, bubble inkjet, drop-on-demand inkjet, and acoustic inkjet. Printheads for such printing methods are commercially available from Hewlett-Packard Corporation, Palo Alto, CA and Lexmark International, Lexington, KY (thermal inkjet); Domino Printing Sciences, Cambridge, UK (continuous inkjet); and Trident International, Brookfield, CT, Epson, Torrance, CA, Hitachi Data Systems Corporation, Santa Clara, CA, Xaar PLC, Cambridge, UK, Spectra, Lebanon, NH, and Idanit Technologies, Ltd., Rishon Le Zion, Israel (piezo inkjet).

Examples of a suitable inkjet printhead models include the NOVA series such as the NOVA-Q printhead commercially available from Spectra Inc., and the XJ 128 series such as the X7128-200 printhead commercially available from Xaar PLC. When using the XJ128-200 printhead, the fluid solution 18 may be coated on the adhesive layer 12 by piezoelectrically driving the printhead at 1.25 kilohertz (kHz) and 35 volts (V), with a printing resolution of 300x300 dots-per-inch (dpi). This generates drops with nominal volumes of about 70 picoliters (pL).

Based on the printing resolution, the percent of the surface 16 covered (i.e., the pixel coverage), and the concentration of the biological active in the fluid solution 18, the concentration of the biological active (Concentration$_{B.A.}$) applied on the adhesive layer 12 may be determined as follows:

$$\text{Concentration}_{B.A.} = \left(\frac{\#\,of\,Drops}{(Inch)^2}\right)\left(\frac{\%Coverage}{100}\right)\left(\frac{Volume}{Drop}\right)(Density_{F.S})\left(\frac{Wt\%_{B.A.}}{100}\right)$$

The ($\#of Drops/Inch^2$) is the number of print pixels in a square inch of the substrate and is based on the selected printing resolution, and the (%Coverage/100) is the fraction of the surface 16 that is printed on. For example, with a printing resolution of 300x300 dpi and a 100% surface coverage of the surface 16, a total of 90,000 drops of the fluid solution 18 are deposited per square inch of the adhesive layer 12. By this definition, the percent coverage may be greater than 100%, where a fraction of the pixels are double printed as the printhead executes multiple passes over the article. For example, with a printing resolution of 300x300 dpi and a 200% surface coverage of the surface 16, a total of 180,000 drops of the fluid solution are deposited per square inch of the surface 16, where 90,000 drops are deposited in the first pass of the printhead, and another 90,000 drops are deposited over the first set of drops in a second pass.

The (Volume/Drop) is the nominal volume of the drops generated by the selected printhead (e.g., 70 pL is the drop volume typically generated by the XJ 128-200 printhead). The (Density$_{F.S.}$) is the average density of the fluid solution 18 and the (Wt%$_{B.A.}$/100) is the weight percent concentration of the biological active in the fluid solution 18 prior to inkjet printing.

The percentage surface coverage of the fluid solution 18 inkjet printed onto the surface 16 may vary as individual needs may require. The percentage required generally depends upon the composition of the fluid solution 18, including the biological active, the activity level of the selected biological active, and the level of biological activity desired. Examples of suitable percentage surface coverages of the fluid solution 18 inkjet printed onto the surface 16 range from about 1% to about 500%.

Based on a printing resolution of 300x300 dpi, a fluid solution 18 containing 1.0% silver oxide as the biological active, which is inkjet printed at a 100% surface coverage onto the surface 16 of the adhesive layer 12 provides about 0.06 milligrams/inch$^2$ (mg/inch$^2$) (about 93 milligrams/meter$^2$) of the silver oxide. This concentration of silver oxide is significantly lower than concentrations of silver reported in conventional antimicrobial articles. However, despite the low concentration, the article 10 exhibits effective antimicrobial activity to reduce the risk of infections.

Inkjet printing also allows for the creation of indicia and graphics on the surface 16 of the adhesive layer 12. As such, the pattern that the fluid solution 18 is inkjet printed onto the surface 16 may also convey textual and graphical messages. In one embodiment, the messages may be visually observable through the use of pigments or dyes contained in the fluid solution 18, which remain concentrated on or near the surface 16 when the fluid solution 18 substantially dries. Preferably, however, the biological active itself provides coloration for the messages on the surface 16. For example, silver-containing compounds, such as silver oxide, are clear when in the fluid solution, but turn a dark brown color when dried. This precludes the need for additional colorants to render the inkjet printed patterns visually observable. Examples of suitable messages include company logos, instructions for use of the article, brand names, and designs for aesthetic appearance.

Spray atomization deposition operates by emitting the fluid solution 18 through an air impingement nozzle or air stripping nozzle to atomize the fluid solution 18 to some degree. The atomized fluid solution 18 is then directed onto the adhesive layer 12. While FIG. I shows the droplets of the fluid solution 18 as being disposed in a generally uniform pattern on the surface 16 (which is typical of inkjet printing), spray atomization deposition generally provides a more random pattern of droplets.

An example of suitable spray atomization deposition systems include commercially available spray heads and bodies, such as those from Spraying Systems Co., Wheaton, IL. The spray heads may also include fan spray adaptations to fan out the primary atomization sources for creating elliptical patterns. Suitable operating conditions include spraying the fluid solution on the surface 16 of the adhesive layer 12 with a volumetric flow rate of about 5 milliliters/minute (mL/min), a web speed of about 15 feet/minute (about 4.6 meters/minute), an atomizer nozzle setting of about 23 pounds/inch$^2$ (psi) (about 159 ki lopascals (kpa)), and a fan nozzle setting of about 20 psi (about 138 kpa).

The spray heads generate droplets with diameters ranging from 2 micrometers to 20 micrometers. After the fluid solution 18 dries; the remaining dried droplets on the adhesive layer 12 exhibit diameters ranging up to 30 micrometers due to agglomerated droplets. Based on the concentration of the biological active in the fluid solution 18, the concentration of the biological active (Concentration$_{B.A.}$) sprayed on the adhesive layer 12 may be determined as follows:

$$\text{Concentration}_{B.A.} = (\%\text{SurfaceArea}_{F.S.})\left(\frac{\text{Volume}}{\text{Area}}\right)(\text{Density}_{F.S.})\left(\frac{\text{Wt\%}_{B.A.}}{100}\right)$$

The percent surface area of the fluid solution 18 (%SurfaceArea$_{F.S.}$) is the ratio of the total surface area of the fluid solution 18 droplets to the surface area of the surface 16, as physically viewed with via digital microscopy. The fluid solution 18 droplets are digitally shown as dark drops on a clear background. As such, the total area of the dark regions and the total area of the clear regions may be compared to provide the ratio. Using the above-discussed operating conditions, the percent area coverage of the biological active molecules typically range from 4.9% to 6.5% of the surface 16.

The (Volume/Area) is a conversion of the surface area of the fluid solution 18 droplets to the volume of the fluid solution 18 droplets. The (Density$_{F.S.}$) is the average density of the fluid solution 18 and the (Wt%$_{B.A.}$/100) is the weight percent concentration of the biological active in the fluid solution 18 prior to spraying.

The fluid solution 18 may also be deposited on the surface 16 of the adhesive layer 12 through separate non-contact deposition systems, such as a plurality of inkjet printing systems. For example, a first inkjet printing system may print a first fluid solution 18 containing a first biological active, and simultaneously or subsequently, a second inkjet printing system may print a second fluid solution 18 containing a second biological active. This is particularly useful for coating multiple biological actives on the same surface 16, where the biological actives are incompatible in a single fluid solution 18. The small drop sizes and the rapid drying of the fluid solutions 18 obtainable by non-contact deposition, reduces the risk of adverse interactions between the first and second biological actives.

The fluid solution 18 may also be deposited by non-contact deposition in a concentration gradient with multiple passes of the non-contact deposition system. For example, a first pass could be contain a high concentration of the biological active, and a subsequent pass could contain a low concentration of the same or a different biological active. This is beneficial for controlling the delivery of the biological active. Moreover, the fluid solution may be deposited in a manner such that the biological active is concentrated in certain areas of the surface 16. For example, the concentration of the biological active may be greater at the central regions of the surface 16 of the article 10, and less at the periphery. This allows lower concentrations of expensive biological actives to be used.

The fluid solution 18 of the present invention desirably exhibits a sufficiently low viscosity to be coated by non-contact deposition. The desired viscosity will generally depend on the non-contact deposition technique used. For example, for inkjet printing, the fluid solution 18 desirably exhibits a viscosity below 30 centipoise (i.e., 30 milliPascal-seconds), preferably below 25 centipoise, and more preferably below 20 centipoise at the desired inkjetting temperature (typically from 25°C to 65°C). However, the optimum viscosity characteristics for the fluid solution 18 will depend primarily upon the inkjetting temperature and the type of inkjet system used. For piezo inkjet applications, suitable viscosities for the fluid solution 18 range from 3 to 30 centipoise, preferably from 10 to 16 centipoise, at temperatures ranging from 25°C to 65°C.

After the fluid solution 18 is applied to the surface 16 of the adhesive layer 12, the fluid solution 18 is allowed to substantially dry. The fluid solution 18 may be allowed to dry in a variety of manners, and may depend on the composition of the fluid solution 18 and the non-contact deposition technique used. In general, rapid drying further reduces the extent that the fluid solution 18 diffuses into the adhesive layer 12.

The non-contact deposition techniques discussed above deposit small drop volumes of the fluid solution 18 on the surface 16 of the adhesive layer 12 (e.g., 70 pL for inkjet printing). As such, the drops generally exhibit large surface areas, which allow the fluid solution 18 to rapidly dry upon application. After non-contact deposition, the article 10 may be held at room temperature (25°C) for a period of time to allow the fluid solution 18 to substantially dry. The period of time will depend on the amount of fluid solution 18 applied to the surface 16 and the composition of the fluid solution 18 (e.g., 30 minutes to 48 hours). The rate of drying may alternatively be increased by holding the article 10 at an elevated temperature (e.g., in an oven at 150°C) for a period of time to allow the fluid solution 18 to substantially dry (e.g., 5 to 10 minutes). Inline drying may also be used, and is particularly useful for webline coating operations. Upon drying, the biological active and other components of the fluid solution 18 that did not volatilize remain concentrated on or near the surface 16 of the adhesive layer 12. A variety of factors, such as the volatility of the fluid solution 18, the volatility of the biological active, heat stability of the biological active, the type of drying oven, the air flow volume, and the degree of impingement may influence the drying time and temperature required to evaporate the fluid solution 18.)

After drying, suitable concentrations of the biological active on the adhesive layer 12 include concentrations of less than 1.0 mg/inch$^2$ (1.55 grams/meter$^2$), preferably less than 0.5 mg/inch$^2$ (0.78 grams/meter$^2$), and more preferably less than 0.1 mg/inch$^2$ (0.16 grams/meter$^2$). As discussed above, the low concentration of the biological active allows the adhesive layer 12 to retain desirable physical properties and antimicrobial activity levels. As such, the article 10 is capable of releasing effective levels of biological actives while retaining adhesive properties during use. After the article 10 is prepared pursuant to the present invention, the adhesive layer 12 desirably exhibits a peel strength that is at least 70%, preferably at least 80%, and more preferably at least 90%, of the uncoated peel strength exhibited by the adhesive layer 12. As used herein, peel strength is determined pursuant to ASTM D3330 using a Thwing-Albert Tensile Tester, commercially available from Thwing-Albert Instrument Co., Philadelphia, PA, with a test surface consisting of a #302 AISI stainless steel annealed surface.

The low concentration of the biological active on or near the surface 16 also allows a low concentration of the biological active to be used in the fluid solution 18. This provides an economic benefit by reducing material costs required to prepare the article 10.

SUITABLE MATERIALS

In addition to the biological active, the fluid solution 18 of the present invention may also include a carrier solvent, where the biological active is substantially dissolved or dispersed within the carrier solvent to obtain an adequate viscosity for non-contact deposition. Examples of suitable carrier solvents include aqueous and non-aqueous solvents such as water, propylene glycol, ethylene glycol, glycerol, methanol, ethanol, isopropanol, and combinations thereof. While referred to as a "solution", the fluid solution 18 may be a dispersion, an emulsion, a solution, and combinations thereof.

The fluid solution 18 may also include a variety of additional materials to enhance the properties of the fluid solution 18 and/or the biological active. Examples of suitable additional materials include plasticizers, binders, excipients, dyes, pigments, surfactants, enhancers, and combinations thereof.

Suitable surfactants for use in the fluid solution 18 are preferably nonionic, and may include surfactants commercially available under the trade designation "PLURONICS" from BASF, Spartanburg, SC; surfactants commercially available under the trade designation "brim" from Imperial Chemical Industries PLC, London, UK; polyethylene oxide and polypropylene oxide copolymers; polyoxyethylene stearyl ethers; polyoxyethylene lauryl ethers; dioctyl sodium sulfosuccinates; alkylpolyglucosides; polyglyceryl esters; dioctylsulfosuccinates; and combinations thereof. Examples of suitable concentrations of the surfactants in the fluid solution range from 1.0% to 20.0% by weight, based on the total weight of the fluid solution 18.

Enhancers may be used to increase the biological activity of certain biological actives (e.g., fatty acid monoesters, fatty acids, and halogenated phenolic compounds such as triclosan). Examples of suitable enhancers include chelating agents such as ethylenediaminetetraacetic acid (EDTA) and salts thereof; organic acids such as lactic acid, tartaric acid, adipic acid, succinic acid, citric acid, ascorbic acid, malic acid, mandelic acid, acetic acid, sorbic acid, benzoic acid, and salicylic acid; alcohols such as ethanol, isopropanol, and long chain alcohols, such as octyl alcohol and decyl alcohol; and combinations thereof. Examples of suitable concentrations of the enhancers in the fluid solution range from 1.0% to 20.0% by weight, based on the total weight of the fluid solution 18.

As discussed above, the fluid solution 18 of the present invention may include a variety of different biological actives, such as antimicrobials, antibiotics, antifungals, antivirals, and antiseptics. The concentration of the biological active in the fluid solution 18 desirably is such that the concentration of the biological active applied to the surface 16 of the adhesive layer 12 is therapeutically effective. As such, the concentration of the biological active in the fluid solution 18 will vary depending on a variety of factors, such as the type of biological active used, the design of the article 10, the condition to be treated, and the length of time the article 10 will be used. Generally the concentration of the biological active in the fluid solution 18 ranges from 0.01% to 50.0% by weight, based on the total weight of the fluid solution 18.

Examples of suitable biological actives in the fluid solution 18 include metal-ion forming compounds (e.g., silver-containing compounds, zinc-containing compounds, copper-containing compounds, gold-containing compounds, and platinum-containing compounds), fatty acid monoesters, polyhexamethylenebiguanide, chlorhexidine, triclosan, peroxides, iodines and complexes thereof (e.g., iodophores), derivatives thereof, and combinations thereof. Additional biological actives that are suitable for use with the present invention include medicinal ingredients disclosed in Cantor et al., U.S. Patent Application No. 10/242,065, entitled "Non-Contact Printing Method for Making a Medical Pressure Sensitive Adhesive Article".

The silver-containing compounds suitable for the biological active include compounds that are soluble in aqueous solvents (e.g., silver nitrate) and sparingly soluble silver-containing (SSSC) compounds, which are disclosed in the concurrently filed patent application, attorney docket no. 59862US002, entitled. "Silver-Releasing Articles and Methods of Manufacture" (referred to herein as "the 59862US002 application"). Silver-containing compounds impart antimicrobial activity to a surface with minimal risk of developing bacterial resistance. The antimicrobial activity of silver is believed to be due to free silver ions or radicals, where the silver ions kill microbes by blocking the cell respiration pathway, by attaching to the cell DNA and preventing replication, and by disruption of the cell membrane. Silver ions are also rarely associated with microbial resistance and do not exhibit significant negative effects on human cells. As such, systematic use of silver-containing compounds generally does not generate concerns in the medical field over antibiotic-resistant bacteria.

The silver-containing compounds suitable for the biological active provide antimicrobial activity by a sustained release of silver ions from the adhesive layer 12 when in contact with moist environments, such as a wound bed. Examples of suitable silver-containing compounds include silver oxide, silver sulfate, silver acetate, silver chloride, silver lactate, silver phosphate, silver stearate, silver thiocyanate, silver proteinate, silver carbonate, silver nitrate, silver sulfadiazine, silver alginate, and combinations thereof. An example of particularly suitable silver-containing compounds include silver oxides, silver carbonates, and silver acetates. Examples of suitable concentrations of the silver-containing compound in the fluid solution 18 range from 0.1% to 15.0% by weight, based on the total weight of the fluid solution 18. Examples of particularly suitable concentrations of the silver-containing compound in the fluid solution 18 range from 1.0 % to 5.0% by weight, based on the total weight of the fluid solution 18.

with regards to silver oxide, a variety of Valence states of the silver oxide may be used (e.g., where the oxidation state is silver (II) oxide or silver (III) oxide). The valence state of the silver oxide concentrated on or near the surface 16 of the adhesive layer 12 may be determined by depositing a silver oxide of a given valence state (e.g., $Ag_2O$, $AgO$, $Ag_2O_3$, $Ag_2O_4$). Alternatively, the valence state of the silver oxide may be increased by including an oxidizing agent to the fluid solution of the present invention, or applying an oxidizing agent to the adhesive layer 12 after applying the fluid solution 18 to the surface 16 by non-contact deposition. Examples of suitable oxidizing agents include hydrogen peroxide, alkali metal persulfates, permanganates, hypochlorites, perchlorates, nitric acid, and combinations thereof. An example of a suitable alkali metal persulfate includes sodium persulfate as discussed in Antelman, U.S. Patent No. 6,436,420.

SSSC compounds, such as silver oxides and select silver salts, exhibit low solubility in aqueous carrier solvents. As such, SSSC compounds are difficult to directly disperse or dissolve in solutions. While this presents an issue for obtaining the fluid solution 18 with such SSSC compounds, the low solubility renders the SSSC compounds excellent sources for slow and sustained release of silver ions.

To accommodate for the low solubility of the SSSC compounds, the fluid solution 18 may also include ammonium-containing compounds. The ammonium-containing compounds complex with the SSSC compounds to substantially dissolve the SSSC compounds in an aqueous carrier solvent. This allows the fluid solution 18 to include SSSC compounds while also exhibiting adequate viscosities for non-contact deposition. Depending on the SSSC compound used, the SSSC compound may readily dissolve in the aqueous carrier solvent at room temperature when mixed with the ammonium-containing compound. If not, mechanical action such as stirring over time and/or heat may be required to aid the dissolution.

Examples of suitable ammonium-containing compounds include ammonium salts such as ammonium pentaborate, ammonium acetate, ammonium carbonate, ammonium chloride, ammonium peroxyborate, ammonium tertraborate, triammonium citrate, ammonium carbamate, ammonium bicarbonate, ammonium malate, ammonium nitrate, ammonium nitrite, ammonium succinate, ammonium sulfate, ammonium tartarate, and combinations thereof. The concentration of the ammonium-containing compound in the fluid solution 18 is desirably the minimum required to dissolve the SSSC compound used. Examples of suitable concentrations of the ammonium-containing compound in the fluid solution 18 range from about 1.0% to about 25% by weight, based on the total weight of the fluid solution 18.

The silver-containing compounds, once applied to the adhesive layer 12, are desirably stable to at least one of the following types of radiation: Visible light, ultraviolet light, electron beam, and gamma ray sterilization. In certain embodiments, the silver-containing compounds are stable to visible light, such that the silver-containing compounds do not darken upon exposure to visible light. Such silver-containing compounds are useful in medical articles, particularly wound dressings and wound packing materials, although a wide variety of other articles may be coated with the silver-containing compounds.

An example of particularly suitable materials for the fluid solution 18 of the present invention include silver oxide, ammonium carbonate, and an aqueous carrier solvent, such as water. While not wishing to be bound by theory, it is believed that the silver oxide and the ammonium carbonate complex to dissolve the silver oxide in the aqueous carrier solvent. The complexing creates a silver ammonium carbonate compound. The fluid solution 18 is then applied to the surface 16 of the adhesive layer 12 by non-contact deposition. During the non-contact deposition, a portion of the ammonium carbonate readily evaporates because of the large surface area of the deposited fluid solution 18.

Because the fluid solution 18 exhibits low solubility with the adhesive layer 12, the fluid solution minimally diffuses into the adhesive layer 12 and remains on or near the surface 16. Moreover, as the fluid solution 18 dries, silver oxide is reformed on the adhesive layer 12. This is believed to be due to the decomplexation of the silver ammonium carbonate compound into silver oxide, ammonia, carbon dioxide, and water. The ammonia, carbon dioxide, and water then evaporate. The decomplexation of the silver oxide is observable by a color change. Prior to drying, the fluid solution 18 is substantially colorless. However, after drying, the residual portion of the fluid solution 18 turns dark brown, which is a typical characteristic of silver oxide. As such, after non-contact deposition, the ammonium carbonate and the water are removed, leaving silver oxide concentrated on or near the surface 16 of the adhesive layer 12.

Silver acetate also exhibits low solubility with aqueous carrier solvents, which also presents an issue for applying the silver acetate on the adhesive layer 12 by non-contact deposition. Accordingly, the fluid solution 18 may also include a dispersant to complex with the silver acetate. Examples of suitable dispersants are the same materials as the suitable surfactants discussed above. The acetate component of the silver acetate compound exists as a counter ion in association with the silver-dispersant adduct. This creates a stable dispersion of the silver acetate in the aqueous carrier solvent that exhibits a low viscosity to allow application by non-contact deposition. Additionally, an ammonium-containing compound may also be used with the dispersant to complex with the silver acetate in the same manner as discussed above for silver oxide. This further increases the solubility of the silver acetate in the aqueous carrier solvent.

Fatty acid monoesters suitable for the biological active are desirably considered food grade and recognized as safe (GRAS) by the U. S. Food and Drug Administration (FDA). Such fatty acid monoesters may be derived from $C_8$ to $C_{12}$ fatty acids such as glycerol monoesters of caprylic acid, capric acid, and lauric acid; propylene glycol monoesters of caprylic acid, capric acid, and lauric acid; fatty acids; and combinations thereof. Examples of suitable fatty acid monoesters include glycerol monolaurate commercially available under the trade designation "LAURICIDIN" from Med-Chem Laboratories, East Lansing, MI; glycerol monocaprylate commercially available under the trade designation "POEM M-100" from Riken Vitamin Ltd., Tokyo, Japan; glycerol monocaprate commercially available under the trade designation "POEM M-200" from Riken Vitamin Ltd.; propylene glycol monolaurate, propylene glycol monocaprylate, and propylene glycol monocaprate, all commercially available from Uniquema International, Chicago, IL; and combinations thereof.

Examples of suitable concentrations of the fatty acid monoester in the fluid solution 18 range from 1.0% to 30.0% by weight, based on the total weight of the fluid solution 18. Examples of particularly suitable concentrations of the fatty acid monoester in the fluid solution 18 range from 5.0% to 20.0% by weight, based on the total weight of the fluid solution 18.

The fluid solution 18 may also include an enhancer and a surfactant for use with the fatty acid monoesters, as discussed in Andrews et al., PCT Application No. WO00/71183, entitled "Antimicrobial Articles", and in Andrews et al., PCT Application No. WO01/43549, entitled "Fruit, Vegetable, and Seed Disinfectants". Examples of suitable enhancers and surfactants for use with the fatty acid monoester include the suitable enhancers and the suitable surfactants for use in the fluid solution 18, as described above. Preferably, the fatty acid monoester, the enhancer, and the surfactant are substantially dissolved in an aqueous or non-aqueous carrier solvent for non-contact deposition.

A particularly suitable combination of biological actives for use with the present invention include a SSSC compound, such as silver oxide, and a fatty acid monoester. The fatty acid monoester is rapidly released upon exposure to moisture from a wound bed, which provides fast antimicrobial activity to prevent bacterial infections. In contrast, the low solubility of the SSSC compound with the moisture causes the silver ions to release at a slower rate. This provides a slower and sustained antimicrobial activity to the wound site relative to the fatty acid monoester. As such, the combined use of the SSSC compound and the fatty acid monoester provides for a two-tiered synergistic antimicrobial activity.

In general, however, fatty acid monoesters and SSSC compounds such as silver oxides are incompatible in a single fluid solution. As such, fatty acid monoesters and SSSC compounds generally may not be applied to the adhesive layer 12 through a single non-contact deposition technique. Nonetheless, as discussed above, multiple non-contact deposition systems may be used to simultaneously or sequentially deposit two fluid solutions 18 on the adhesive layer 12. One fluid solution 18 may contain the fatty acid monoester and the other fluid solution 18 may contain the SSSC compound. For example, a fatty acid monoester and a silver oxide may be inkjet printed onto the adhesive layer 12 with separate inkjet printheads prior to the drying step. Alternatively, an inkjet system may be used to deposit the SSSC compound and a spraying system may be used to deposit the fatty acid monoester (or vice versa). This allows both the fatty acid monoester and the silver oxide to remain concentrated on or near the surface 16 of the adhesive layer 12, with minimal interactions between the two biological actives.

Examples of suitable chlorhexidine materials for the biological active include chlorhexidine, chlorhexidine salt derivatives such as chlorhexidine digluconate (typically referred to as chlorhexidine gluconate or CHG) and chlorhexidine acetate, and combinations thereof. Examples of suitable concentrations of the chlorhexidine materials in the fluid solution 18 range from 1.0% to 40.0% by weight, based on the total weight of the fluid solution 18. Examples of particularly suitable concentrations of the chlorhexidine materials in the fluid solution 18 range from 5.0% to 20.0% by weight, based on the total weight of the fluid solution 18.

ADHESIVE ARTICLES

The article 10 represents a suitable article that may be prepared with a biological active pursuant to the present invention. Preferably, the articles (e.g., the article 10) are adhesive medical articles, such as adhesive wound dressings. Examples of suitable adhesive medical articles include adhesive wound dressings under the trade designation "TEGADERM" Dressings, which are commercially available from 3M Corporation, St. Paul, MN.

As shown in FIG. 1, the backing substrate 14 of the article 10 generally defines the bulk of the article 10 (e.g., a gauze bandage for a wound dressing). The adhesive layer 12 is a layer of an adhesive material disposed on the backing substrate 14 to adhere the article 10 to a surface, such as the skin of a patient.

Examples of suitable materials for the backing substrate 14 include fabric, non-woven or woven polymeric webs, knits, polymer films, hydrocolloids, foam, metallic foils, paper, gauze, natural or synthetic fibers, cotton, rayon, wool, hemp, jute, nylon, polyesters, polyacetates, polyacrylics, alginates, ethylene-propylene-diene rubbers, natural rubber, polyesters, polyisobutylenes, polyolefins (e.g., polypropylene polyethylene, ethylene propylene copolymers, and ethylene butylene copolymers), polyurethanes (including polyurethane foams), vinyls including polyvinylchloride and ethylene-vinyl acetate, polyamides, polystyrenes, fiberglass, ceramic fibers, elastomers, thermoplastic polymers, and combinations thereof. Such materials are typically used as backing substrates in a variety of conventional medical products.

The adhesive layer 12 is preferably a PSA. Examples of suitable materials for the adhesive layer 12 include PSA's based on acrylates, polyurethanes, silicones, rubber based adhesives (including natural rubber, polyisoprene, polyisobutylene, and butyl rubber), and combinations thereof. Examples of suitable acrylates include polymers of alkyl acrylate monomers such as methyl methacrylate, ethyl methacrylate, n-butyl methacrylate, methyl acrylate, ethyl acrylate, n-butyl acrylate, iso-octyl acrylate, iso-nonyl acrylate, 2-ethyl-hexyl acrylate, decyl acrylate, dodecyl acrylate, n-butyl acrylate, hexyl acrylate, and combinations thereof.

As discussed above, to prevent the biological active from diffusing into the adhesive layer 12, the fluid solution 18 and the adhesive layer 12 of the article 10 exhibit low solubilities. Typically, the materials of the adhesive layer 12 exhibit Hildebrand solubility parameters of about 20.0 $MPa^{1/2}$ (about 9.8 $(calories/cm^3)^{1/2}$) or less. This allows the biological active to remain concentrated on or near the surface 16 of the adhesive layer . 12 after the fluid solution 18 is applied by non-contact deposition.

An example of particularly suitable materials for the adhesive layer 12 include silicone-based adhesives, which exhibit several beneficial properties over traditional PSA's used in wound care applications. For example, silicone-based adhesives may be formulated to offer good skin adhesion characteristics, offer excellent conformability, and provide a gentle release from the skin and wound site. Typically, silicone-based adhesives are formed from the reaction of a polysiloxane gum and a resin as a two part system, one part hindered system to prevent premature reaction, or even as a hot melt system. Examples of suitable silicone-based adhesives include polydiorganosiloxane-based adhesives; adhesives commercially available under the trade designation "SILASTIC 7-6860" Biomedical Grade Adhesive from Dow Corning Corp., Midland, MI; adhesives disclosed in Sherman et al., U.S. Patent No. 6,407,195; and combinations thereof.

The article 10 may also include a liner (not shown) that is disposed on the adhesive layer 12 and the biological active, opposite the backing substrate 14, to protect the adhesive layer 12 prior to use. Liners which are suitable for use with the article 10 may be made of materials such as kraft papers, polyethylene, polypropylene, polyester, and combinations thereof. The liners are preferably coated with compositions containing release agents, such as polymerized fluorochemicals or silicones. The low surface energy of the liner provides for an easy removal from the surface 16 of the adhesive layer 12 without substantially affecting the biological active that is concentrated on or near the surface 16.

PROPERTY ANALYSIS AND CHARACTERIZATION PROCEDURES

Various analytical techniques are available for characterizing the sealant materials of the present invention. Several of the analytical techniques are employed herein. An explanation of these analytical techniques follows.

Solubility Test

Solubility between the fluid solutions of the present invention and the adhesive layers of articles being coated were quantitatively determined using Hildebrand solubility parameters. For a given sample, the Hildebrand solubility parameter of the fluid solution was compared to the Hildebrand solubility parameter of the corresponding adhesive layer that the fluid solution was applied to. The closer the Hildebrand solubility, parameters between the fluid solution and the adhesive layer were, the more soluble and compatible they were. Conversely, the further apart the Hildebrand solubility parameters between the fluid solution and the adhesive layer were, the less soluble they were.

The Hildebrand solubility parameter of a mixture of multiple substances was based on the weighted average of the Hildebrand solubility parameters of the individual substances, based on the total weight of the mixture. For example, a fluid solution of 1.0% silver (I) oxide and 5.0% ammonium carbonate in water consists primarily of water (i.e., 94% water). As such, the Hildebrand solubility parameter of the fluid solution would be comparable to the Hildebrand solubility parameter of water (i.e, 47.9 MPa$^{1/2}$ or (23.4 cal/cm$^3$)$^{1/2}$).

Zone of Inhibition Test

Antimicrobial performance was quantitatively determined for adhesive articles prepared pursuant to the present invention using a zone of inhibition test, which was performed by the following method. A solution of staphylococcus aureus (A.T.C.C. 25923) was prepared at a concentration of $1 \times 10^9$ colony forming units per milliliter (ml) in Phosphate Buffered Saline using a 0.5 McFarland Equivalence Turbidity Standard. Bacterial lawns were prepared by dipping a sterile cotton applicator into the solution and swabbing a dry surface of a trypticase soy agar plate in three different directions. Three 7-millimeter (mm) diameter discs for each sample were then placed onto the plate and pressed firmly against the agar with sterile forceps to ensure a complete contact with the agar.

The plate was held in a refrigerator at 4°C for three hours and then incubated at 36°C $\pm$ 1°C for 24 hours. A measurement was then made of the diameter of the area around each sample (including the area under the 7-mm diameter sample disc) where inhibited growth and/or no growth was observed. The zone of inhibition was measured using primary and/or secondary zone of inhibitions. The primary zone of inhibition was defined as the diameter of the area that no growth was observed (including the area under the 7-mm diameter sample disk). The secondary zone of inhibition was defined as the diameter of the area that inhibited growth was observed (including the area of the primary zone of inhibition).

Peel Strength Test

Adhesive strengths of the adhesive articles prepared pursuant to the present invention were quantitatively determined pursuant to the ASTM D3330 using a Thwing-Albert Tensile Tester, commercially available from Thwing-Albert Instrument Co., Philadelphia, PA. The test surface consisted of a #302 AISI stainless steel annealed surface, which was cleaned with a 50/50 mixture of isopropanol and heptane. The samples were pulled at a 180° angle with a crosshead speed of 300 millimeters/minute and a gauge length 125 mm. The recorded adhesive strength was the average of six measurements.

Finger Tack Test

Adhesive strengths of the adhesive articles prepared pursuant to the present invention were qualitatively determined for skin adhesion characteristics by applying moderate finger pressure to the adhesive articles and pulling the finger away. This technique provides a good initial screening for adhesion quality and feel. Adhesive strengths that are too high are undesirable as they may be difficult to remove from a wound site without aggravating the wound and causing pain to the patient. Similarly, adhesive strengths that are too low are also undesirable as the adhesive articles may not remain adhered to the wound site. Finger tack was considered "good" if the adhesive article provided a comfortable level of adhesion to the finger, and was readily removable without requiring a significant amount of force.

EXAMPLES

The present invention is more particularly described in the following examples that are intended as illustrations only, since numerous modifications and variations within the scope of the present invention will be apparent to those skilled in the art. Unless otherwise noted, all parts, percentages, and ratios reported in the following examples are on a weight basis, and all reagents used in the examples were obtained, or are available, from the chemical suppliers described below, or may be synthesized by conventional techniques.

The following compositional abbreviations are used in the following Examples:

| | |
|---|---|
| "Silver (I) oxide": | A silver oxide ($Ag_2O$) with a formula weight of 231.7, commercially available from Alfa Aesar, Ward Hill, MA. |
| "Silver (II) oxide": | A silver oxide (AgO) with a formula weight of 123.9, commercially available from Alfa Aesar, Ward Hill, MA. |
| "Silver acetate": | A silver acetate ($AgCH_3CO_2$) with a formula weight of 166.9, commercially available from Matheson, Coleman, and Bell Co., Norwood, OH. |
| "Silver sulfate": | A silver sulfate ($Ag_2SO_4$) with a formula weight of 311.8, commercially available from Mallinckrodt Chemical, St. Louis, MO. |

| | |
|---|---|
| "Silver nitrate": | A silver nitrate ($AgNO_3$) with a formula weight of 169.9, commercially available from Alfa Aesar, Ward Hill, MA. |
| "CHG": | 20% chlorhexidine gluconate by weight in water, commercially available from Xttrium Laboratories, Inc., Chicago, IL (the weight percents of CHG listed in the Examples below are based on the solid weight of the CHG, and do not include the water). |
| "Lauricidin": | a glycerol monolaurate fatty acid monoester, commercially available under the trade designation "LAURICIDIN" from Med-Chem Laboratories, East Lansing, MI. |
| "Ammonium carbonate": | An ammonium carbonate (($NH_4)_2CO_3$) with a formula weight of 96.1, commercially available from Sigma-Aldrich Chemical Company, Saint Louis, MO. |
| "Ammonium acetate": | An ammonium acetate ($NH_4CH_3CO_2$) with a formula weight of 77.1, commercially available from Sigma-Aldrich Chemical Company, Saint Louis, MO. |
| "ammonia": | 28% ammonia ($NH_3$) with a formula weight of 17.0 in water, commercially available from Sigma-Aldrich Chemical Company, Saint Louis, MO. |
| "Brij 700": | A polyoxyethylene stearyl ether, commercially available under the trade designation "BRIJ 700" from Imperial Chemical Industries PLC, London, UK. |
| "Jeffamine T-403": | A polyether triamine epoxy curing agent, commercially available under the trade designation "JEFFAMINE T-403", from Huntsman Corporation, Houston, TX. |
| "DOSS surfactant": | A dioctylsulfosuccinate (DOSS) surfactant, commercially available from Alfa Aesar, Ward Hill, MA. |
| "Salicylic acid": | A 2-hydroxybenzoic acid ($HOC_6H_8CO_2H$) with a formula weight of 138.1, commercially available from Sigma- Aldrich Chemical Company, Saint Louis, MO. |
| "Binder polymer": | 15% binder polymer in water, where the binder polymer consisted of 50/20/20/10 dimethylaminoethyl methacrylate (oxidized) / methyl methacrylate / isobutyl methacrylate/stearyl methacrylate, all commercially available from Sigma-Aldrich Chemical Company, Saint Louis, MO. |
| "Isopropanol": | A 2-propanol ($(CH3)CHOH$) with a formula weight of 60.1, commercially available from EM Science, Gibbstown, NJ. |
| "Tegaderm": | A wound care product with a polyurethane backing and a press-sensitive adhesive layer, commercially available under the trade designation "TEGADERM" Dressing from 3M Corporation, St. Paul, MN. |
| "Paper-backed Tegaderm": | A wound care product with a paper backing and a press- sensitive adhesive layer, commercially available under the trade designation "TEGADERM" Dressing from 3M Corporation, St. Paul, MN. |
| "Tegaderm HP": | A wound care product with a polyurethane backing and a high moisture vapor transmissive press-sensitive adhesive layer, commercially available under the trade designation "TEGADERM HP" Dressing from 3M Corporation, St. Paul, MN. |
| "Acticoat 7": | A silver-releasing wound dressing commercially available under the trade designation "ACTICOAT 7", from Westaim Biomedical Corporation, Wakefield, MA. The wound dressing is believed to include about 3 milligrams/inch$^2$ of silver on a high-density polyethylene mesh. |
| "Isooctylacrylate": | An isooctylacrylate monomer, commercially available from Sigma-Aldrich Chemical Company, Saint Louis, MO. |
| "Acrylamide": | An acrylamide monomer, commercially available from Sigma-Aldrich Chemical Company, Saint Louis, MO. |
| "Polyurethane": | A polyurethane, commercially available under the trade designation "ESTATE 58309-022" from Noveon, Inc., Cleveland, OH. |
| "Silastic adhesive": | A silicone pressure sensitive adhesive commercially available under the trade designation "SILASTIC 7-6860" (Parts A and B) Biomedical Grade Adhesive from Dow Corning Corp., Midland, MI. |

Example 1

A fluid solution of 1.0% silver (I) oxide and 5.0% ammonium carbonate in water was prepared by heating the mixture to 60°C and stirring until the silver (I) oxide was dissolved. The fluid solution was inkjet printed at 100% surface coverage onto the adhesive surface of Tegaderm with a "XAAR XJ128-200 printhead". The printhead was piezoelectrically driven at 1.25 kHz and 35 V, with a printing resolution of 300x300 dpi. This generated drops of the fluid solution with nominal volumes of about 70 pL. The sample was then dried in an oven at 150°C for 10 minutes.

### Example 2

The fluid solution of Example I was inkjet printed at 200% surface coverage onto the adhesive surface of Tegaderm and dried, pursuant to the inkjet printing method described in Example 1.

### Example 3

The fluid solution of Example 1 was inkjet printed at 100% surface coverage onto the adhesive surface of Tegaderm, pursuant to the inkjet printing method described in Example 1, except that the coated sample was dried at room temperature (25°C) for 24 hours.

### Example 4

The fluid solution of Example I was inkjet printed at 100% surface coverage onto the adhesive surface of Tegaderm HP and dried, pursuant to the inkjet printing method described in Example 1.

### Example 5

The fluid solution of Example I was inkjet printed at 100% surface coverage onto the adhesive surface ofTegaderm HP, pursuant to the inkjet printing method described in Example 1, except that the coated sample was dried at room temperature (25°C) for 24 hours.

### Example 6

A fluid solution of 2.0% silver (I) oxide and 10.0% ammonium carbonate in water was prepared by stirring the mixture until the silver (I) oxide was dissolved. The fluid solution was inkjet printed at 100% surface coverage onto the adhesive surface of Tegaderm and dried, pursuant to the inkjet printing method described in Example 1.

### Example 7

The fluid solution of Example 6 was inkjet printed at 200% surface coverage onto the adhesive surface ofTegaderm and dried, pursuant to the inkjet printing method described in Example I.

### Example 8

A fluid solution of 3.0% silver (II) oxide and 5.0% ammonium carbonate in water was prepared by stirring the mixture until the silver (II) oxide was dissolved. The fluid solution was inkjet printed at 50% surface coverage onto the adhesive surface of Tegaderm and dried, pursuant to the inkjet printing method described in Example 1.

### Example 9

The fluid solution of Example 8 was inkjet printed at 80% surface coverage onto the adhesive surface ofTegaderm and dried, pursuant to the inkjet printing method described in Example 1.

### Example 10

The fluid solution of Example 8 was inkjet printed at 100% surface coverage onto the adhesive surface of Tegaderm and dried, pursuant to the inkjet printing method described in Example 1.

### Example 11

The fluid solution of Example 1 was inkjet printed at 120% surface coverage onto the adhesive surface ofTegaderm and dried, pursuant to the inkjet printing method described in Example I.

### Example 12

A fluid solution of 2.0% silver (I) oxide and 5.0% ammonium carbonate in water was prepared by heating the mixture to 60°C and stirring until the silver (I) oxide was dissolved. The fluid solution was inkjet printed at 120% surface coverage onto the adhesive surface of Tegaderm and dried, pursuant to the inkjet printing method described in Example 1.

Example 13

A fluid solution of 1.0% silver (II) oxide and 5.0% ammonium carbonate in water was prepared by stirring the mixture until the silver (II) oxide was dissolved. The fluid solution was inkjet printed at 120% surface coverage onto the adhesive surface of Tegaderm and dried, pursuant to the inkjet printing method described in Example 1.

Example 14

A fluid solution of 2.0% silver (II) oxide and 5.0% ammonium carbonate in water was prepared by stirring the mixture until the silver (I) oxide was dissolved. The fluid solution was inkjet printed at 120% surface coverage onto the adhesive surface of Tegaderm and dried, pursuant to the inkjet printing method described in Example I.

Example 15

The fluid solution of Example 8 was inkjet printed at 120% surface coverage onto the adhesive surface of Tegaderm and dried, pursuant to the inkjet printing method described in Example 1.

Example 16

A fluid solution of 1.0% silver acetate, 5.0% ammonium acetate, and 1.5% ammonia in water was prepared by heating the mixture to 60°C and stirring until the silver acetate was dissolved. The fluid solution was inkjet printed at 160% surface coverage onto the adhesive surface of Tegaderm and dried, pursuant to the inkjet printing method described in Example I.

Example 17

The fluid solution of Example 16 was inkjet printed at 160% surface coverage onto the adhesive surface of Tegaderm, pursuant to the inkjet printing method described in Example 1, except that the coated sample was dried at room temperature (25°C) for 24 hours.

Example 18

A fluid solution of 1.0% silver sulfate and 5.0% ammonium acetate in water was prepared by heating the mixture to 70°C and stirring until the silver sulfate was dissolved. The fluid solution was inkjet printed at 160% surface coverage onto the adhesive surface of Tegaderm and dried, pursuant to the inkjet printing method described in Example 1.

Example 19

The fluid solution of Example 18 was inkjet printed at 160% surface coverage onto the adhesive surface ofTegaderm, pursuant to the inkjet printing method described in Example 1, except that the coated sample was dried at room temperature (25°C) for 24 hours.

Example 20

A fluid solution of 1.5% silver acetate, 4.0% Brij 700, and 4.0% Jeffamine T-403 in water was prepared by stirring the mixture until the silver acetate was dispersed. The fluid solution was inkjet printed at 100% surface coverage onto the adhesive surface of Tegaderm and dried, pursuant to the inkjet printing method described in Example 1.

Upon mixing, the fluid solution of Example 20 was transparent with a slight brownish tint, which became darker brown with time. However, after two months at 25°C, no settling of the silver compound was observed and the fluid solution remained transparent.

Example 21

A fluid solution of 20% chlorhexidine gluconate (CHG) in water was inkjet printed at various surface coverages onto an adhesive layer of an article and dried, pursuant to the inkjet printing method described in Example 1. The various surface coverages included 0.1%, 0.2%, 0.5%, 1.0%, 2.0%, 5.0%, and 10.0%.

The coated article exhibited a 2.5 cm x 15.0 cm printing surface for each coating percentage, and contained the adhesive layer in a concentration of 25 grams/meter$^2$ on a 20 micrometer thick polyurethane backing substrate. The adhesive layer consisted of a copolymer of 97/3 isooctylacrylate/acrylamide.

Example 22

The fluid solution of Example 8 was inkjet printed at 100% surface coverage onto an adhesive surface of a silicone pressure sensitive adhesive (PSA) article, pursuant to the inkjet printing method described in Example 1, except that the coated sample was dried in an oven at 150°C for 5 minutes.

The silicone PSA layer was prepared by mixing 30 grams of Part A and 30 grams of Part B ofa Silastic adhesive. The mixed Silastic adhesive was coated onto a 50 micrometer-thick polyester film at a 50 micrometer gap via knife coating. The silicone PSA article was then cured at 100°C for 15 minutes to react the silicone gum and resin to form a silicone PSA layer.

Example 23

The fluid solution of Example 8 was inkjet printed at 100% surface coverage onto the adhesive surface of the silicone PSA article of Example 22, pursuant to the inkjet printing method described in Example 1, except that the coated sample was dried at room temperature (25°C) for 24 hours.

Example 24

A fluid solution of 20.0% Lauricidin, 10.0% salicylic acid, and 10.0% DOSS surfactant in isopropanol was prepared by stirring the mixture until the Lauricidin was dissolved. The fluid solution was inkjet printed at 200% surface coverage onto the adhesive surface of the silicone PSA article of Example 22 and dried, pursuant to the inkjet printing method described in Example 1, except that the coated sample was dried at room temperature (25°C) for 24 hours.

Example 25

A fluid solution of 20.0% CHG in water was inkjet printed at 100% surface coverage onto the adhesive surface of the silicone PSA article of Example 22, pursuant to the inkjet printing method described in Example 1, except that the coated sample was dried at room temperature (25°C) for 24 hours.

Example 26

A fluid solution of 8.0% CHG and 40% binder polymer solution (15.0% binder polymer in water) in water was prepared by stirring the mixture until the CHG was dissolved. The fluid solution was inkjet printed at 100% surface coverage onto the adhesive surface of the silicone PSA article of Example 22, pursuant to the inkjet printing method described in Example 1, except that the coated sample was dried at room temperature (25°C) for 24 hours.

Example 27

The fluid solution of Example 8 was deposited by spray atomization deposition at 20 ml/min onto the adhesive surface of paper-backed Tegaderm with "Coolnozzle 45" spray head with a fan spray adaptation, available from 3M Corporation, St. Paul, MN, and a 1/8VUA-SS body, commercially available from Spraying Systems Co., Wheaton, IL. The atomizer nozzle setting was 23 psi (159 kpa) and the fan nozzle setting was 20 psi (138 kpa). The spray head generated droplets with diameters ranging from 2 micrometers to 20 micrometers. The coated sample was then dried in an oven at 150°C for 10 minutes.

Example 28

A fluid solution of 1.0% silver nitrate in water was prepared by stirring the mixture until the silver nitrate was dissolved. The fluid solution was sprayed onto the adhesive surface of Tegaderm using "Coolnozzle 45" spray head with a fan spray adaptation, available from 3M Corporation, St. Paul, MN, and a 1/8VUA-SS body, commercially available from Spraying Systems Co., Wheaton, IL. The fluid solution was dispensed at 10 psi (69 kpa), the atomizer nozzle setting was 21 psi (145 kpa), the fan nozzle setting was 5 psi (34 kpa), the spray shot was 12 milliseconds, and the spray head was placed 26 centimeters above the adhesive layer of the sample.

Solubility Test for Examples 1-28

The Hildebrand solubility parameters of the fluid solutions and the corresponding adhesive layers of Examples 1-28 were compared pursuant to the above-described method entitled "Solubility Test". The fluid solutions of Examples 1-23 and 25-28 consisted primarily of water, (ranging from 80% to 99% water), which exhibits a Hildebrand solubility parameter of 47.9 MPa$^{1/2}$ ((23.4 cal/cm$^3$)$^{1/2}$). As such, the fluid solutions of Examples 1-23 and 25-28 exhibited high Hildebrand solubility parameters. The fluid solution of Example 24 consisted of 60% isopropanol, which exhibits a Hildebrand solubility parameter of 23.5 MPa$^{1/2}$ (11.5 cal/cm$^3$)$^{1/2}$).

The fluid solutions of Examples 1-20,27, and 28 were applied to adhesive layers of Tegaderm, paper-backed Tegaderm, or Tegaderm HP. The adhesive layers of Tegaderm and paper-backed Tegaderm exhibit a Hildebrand solubility parameter of about 16.0 MPa$^{1/2}$ ((about 7.8 cal/cm$^3$)$^{1/2}$). The adhesive layer of Tegaderm HP exhibits a Hildebrand solubility parameter of about 18.4 MPa$^{1/2}$. ((about 9.0 cal/cm$^3$)$^{1/2}$). These values are substantially lower than the Hildebrand solubility parameters for the fluid solutions of Examples 1-20, 27, and 28.

The fluid solution of Example 21 was applied to an adhesive layer consisting of a 97/3 mixture of isooctylacrylate/acrylamide. Because of the relatively high concentration of the isooctylacrylate, the adhesive layer used for the coated sample of Example 21 exhibited an average Hildebrand solubility parameter of about 16.0 MPa$^{1/2}$ ((7.8 cal/cm$^3$)$^{1/2}$). This is substantially lower than the Hildebrand solubility parameter for the fluid solution of Example 21.

The fluid solutions of Examples 22-26 were applied to a silicone PSA layer. The silicone PSA layer is derived from silicone rubber similar to polydimethylsiloxane, which exhibits a Hildebrand solubility parameter of about 15.5 MPa$^{1/2}$ ((7.6 cal/cm$^3$)$^{1/2}$). This is substantially lower than the Hildebrand solubility parameter for the fluid solution of Examples 22-26.

As shown, the fluid solutions of Examples 1-28 exhibit low solubility with the corresponding adhesive layers. As such, the fluid solutions of Examples 1-28 minimally diffuse into the adhesive layers, allowing the biological actives to remain on or near the surfaces of the adhesive layers.

Zone of Inhibition Testing for Examples 1, 2, 4-16, 18, and 22-27

A zone of inhibition test was performed on the coated samples of Examples 1, 2, 4-16, 18, and 22-27 and on Acticoat 7 (Comparative Example A), pursuant to the above-described method entitled "Zone of Inhibition Test". Table 2 provides the primary and secondary zone of inhibition (ZOI) results for the coated samples of Examples 1, 2, 4-16, 18, and 22-27 and Comparative Example A.

TABLE 2

| Example | Percent by Weight of Biological Active* | Percent Surface Coverage | Primary ZOI (mm) | Secondary ZOI (mm) |
|---|---|---|---|---|
| Example 1 | 1.0% Ag$_2$O | 100% | 10 | 12 |
| Example 2 | 1.0% Ag$_2$O | 200% | 10 | 12 |
| Example 4 | 1.0% Ag$_2$O | 100% | 11 | 13 |
| Example 6 | 2.0% Ag$_2$O | 100% | 10 | 11 |
| Example 7 | 2.0% Ag$_2$O | 200% | 11 | 13 |
| Example 8 | 3.0% AgO | 50% | 10 | None |
| Example 9 | 3.0% AgO | 80% | 11 | None |
| Example 10 | 3.0% AgO | 100% | 11 | 12 |
| Example 11 | 1.0% Ag$_2$O | 120% | 10 | 12 |
| Example 12 | 2.0% Ag$_2$O | 120% | 11 | 13 |
| Example 13 | 1.0% AgO | 120% | 8 ** | 11 |
| Example 14 | 2.0% AgO | 120% | 11 | 14 |
| Example 15 | 3.0% AgO | 120% | 12 | 15 |
| Example 16 | 1.0% AgCH$_3$CO$_2$ | 160% | 10 | 13 |
| Example 18 | 1.0% Ag$_2$SO$_3$ | 160% | 10 | 14 |

(continued)

| Example | Percent by Weight of Biological Active* | Percent Surface Coverage | Primary ZOI (mm) | Secondary ZOI (mm) |
|---|---|---|---|---|
| Example 22 | 3.0% Ag$_2$O | 100% | 9 | 15 |
| Example 23 | 3.0% Ag$_2$O | 100% | 10 | 12 |
| Example 24 | 20.0% Lauricidin | 200% | 13 | None |
| Example 25 | 20.0% CHG | 100% | 21 | None |
| Example 26 | 8.0% CHG | 100% | 19 | None |
| Example 27 | 3.0% AgO | 100% *** | 9 | None |
| Comparative Example A | ---- | ---- | 11 | 12 |

(*) Based on the total weight of the fluid solution
(**) Trace growth was observed under the sample disc.
(***) The sample for Example 27 was coated by atomic spray deposition rather than ink jet printing.

The data provided in Table 2 illustrates the antimicrobial activity exhibited by the coated samples prepared pursuant to the present invention. The coated samples of almost all of the Examples exhibited similar antimicrobial levels to Acticoat 7 (Comparative Example A), which contains 3 mg/inch$^2$ silver. Based on the concentration calculation discussed above for inkjet printing, the coated samples for Examples 1-23 contained 0.06 mg/inch$^2$ to 0.20 mg/inch$^2$ silver, which is substantially less than the concentration of Acticoat 7. As such, the coated samples of Examples 1-23 exhibit effective levels of antimicrobial activity with low concentrations of silver.

The data in Table 2 also illustrates that the coated samples with greater concentrations of silver correspondingly exhibited greater zones of inhibition. This is observable in two manners. First, the coated samples of Examples 8-10 were printed with a fluid solution containing 3.0% silver (II) oxide. However, the percent surface coverages varied (i.e., 50%, 80%, and 100%, respectively). As discussed above, the concentration of silver on the coated samples is proportional to the percent surface coverage. Therefore, the coated sample of Example 10 contained the greatest amount of silver and the coated sample of Example 8 contained the least amount of silver. As shown in Table 2, the zones of inhibition correspondingly follow this trend of increased silver concentration.

Similarly, the coated samples of Examples 11-15 were printed with the same percent surface coverage (i.e., 120%), but with varying silver concentrations. The coated samples of Examples 11 and 12 were printed with fluid solutions containing 1.0% and 2.0% silver (I) oxide, respectively, and the Examples 13-15 were printed with fluid solutions containing 1.0%, 2.0%, and 3.0% silver (11) oxide, respectively. As shown in Table 2, the increasing concentrations of the respective silver oxides corresponds with the increased zone of inhibition.

The coated samples of Examples 25 and 26, which included 20% and 8% CHG, respectively, exhibited the greatest zones of inhibition. This may be attributable to the higher solubility of CHG in the moist agar compared to the SSSC compounds in the moist agar.

Peel Strength Testing for Examples 1-20 and 27

Peel strength tests were performed on the coated samples of Examples 1-20 and 27, and on Tegaderm (Comparative Example B), Tegaderm HP (Comparative Example C), and paper-backed Tegaderm (Comparative Example D), pursuant to the above-described method entitled "Peel Strength Test". Table 3 provides the peel strength results for the coated samples of Examples 1-7 in comparison to Comparative Examples B and C. Table 4 provides the peel strength results for the coated samples of Examples 8-20 in comparison to Comparative Example D. Table 5 provides the peel strength results for the coated sample of Example 27 in comparison to Comparative Example D.

TABLE 3

| Example | Percent by Weight of Biological Active * | Percent Surface Coverage | Adhesion (grams/cm) | Standard Deviation (grams/cm) |
|---|---|---|---|---|
| Example 1 | 1.0% Ag$_2$O | 100% | 149.8 | 11.7 |
| Example 2 | 1.0% Ag$_2$O | 200% | 135.3 | 13.5 |

(continued)

| Example | Percent by Weight of Biological Active * | Percent Surface Coverage | Adhesion (grams/cm) | Standard Deviation (grams/cm) |
|---|---|---|---|---|
| Example 3 | 1.0% $Ag_2O$ | 100% | 123.4 | 9.4 |
| Example 4 | 1.0% $Ag_2O$ | 100% | 185.9 | 12.1 |
| Example 5 | 1.0% $Ag_2O$ | 100% | 159.3 | 26.3 |
| Example 6 | 2.0% $Ag_2O$ | 100% | 143.5 | 9.6 |
| Example 7 | 2.0% $Ag_2O$ | 200% | 145.4 | 11.6 |
| Comparative Example B | --- | ---- | 121.8 | 12.6 |
| Comparative Example C | ---- | ---- | 208.9 | 16.2 |
| (*) Based on the total weight of the fluid solution. | | | | |

TABLE 4

| Example | Percent by Weight of Biological Active* | Percent Surface Coverage | Adhesion (grams/cm) | Standard Deviation (grams/cm) |
|---|---|---|---|---|
| Example 8 | 3.0% AgO | 50% | 155.8 | 6.8 |
| Example 9 | 3.0% AgO | 80% | 165.5 | 22.1 |
| Example 10 | 3.0% AgO | 100% | 172.2 | 9.2 |
| Example 11 | 1.0% $Ag_2O$ | 120% | 212.4 | 19.1 |
| Example 12 | 2.0% $Ag_2O$ | 120% | 181.7 | 16.2 |
| Example 13 | 1.0% AgO | 120% | 162.1 | 12.9 |
| Example 14 | 2.0% AgO | 120% | 169.1 | 7.5 |
| Example 15 | 3.0% AgO | 120% | 161.8 | 13.4 |
| Example 16 | 1.0% $Ag_2CH_3CO_3$ | 160% | 133.8 | 11.7 |
| Example 17 | 1.0% $Ag_2CH_3CO_2$ | 160% | 152.6 | 12.2 |
| Example 18 | 1.0% $Ag_2SO_4$ | 160% | 170.3 | 8.4 |
| Example 19 | 1.0% $Ag_2SO_4$ | 160% | 151.2 | 12.8 |
| Example 20 | 1.0% $Ag_2CH_3CO_2$ | 100% | 165.0 | 18.2 |
| Comparative Example D | --- | -- | 179.1 | 17.7 |
| (*) Based on the total weight of the fluid solution. | | | | |

TABLE 5

| Example | Percent by Weight of Biological Active* | Percent Surface Coverage | Adhesion (grams/cm) | Standard Deviation (grams/cm) |
|---|---|---|---|---|
| Example 27 | 3.0% AgO | 100% ** | 259.6 | 13.4 |

(continued)

| Example | Percent by Weight of Biological Active* | Percent Surface Coverage | Adhesion (grams/cm) | Standard Deviation (grams/cm) |
|---|---|---|---|---|
| Comparative Example D | --- | ---- | 340.4 | 54.7 |

(*) Based on the total weight of the fluid solution.
(**) The sample for Example 27 was coated by atomic spray deposition rather than ink jet printing.

The data provided in Tables 3-5 illustrate the good Adhesion strengths retained by the coated samples of Examples 1-20 and 27. As shown, the adhesive layers retain peel strengths ranging from 75% to greater than 100% of the pre-coated adhesive strengths. For example, the coated samples of Examples 1-3, 6, and 7, which were inkjet printed on the adhesive layer ofTegaderm, exhibited greater peel strengths than the peel strengths of the sample of Comparative Example B (un-coated Tegaderm). Similarly, the coated samples of Examples 4 and 5, which were inkjet printed on the adhesive layer of Tegaderm HP exhibited peel strengths ranging from 76% to 89% of the sample of Comparative Example C (un-coated Tegaderm HP).

The adhesive strengths of the adhesive layers were generally not proportional to the concentration of the biological actives applied. The coated samples of Examples 11-15 did not exhibit substantial differences in peel strengths despite the varying concentrations of silver applied. However, with regards to the coated samples of Examples 8-10, the coated samples with greater silver concentrations actually exhibited greater peel strengths than the coated samples with less silver concentrations.

In general, the majority of the coated samples of Examples 1-20 and 27 exhibited peel strength drops of about 10% or less, compared to the uncoated samples of Comparative Examples B-D. As such, despite having biological actives concentrated on or near the surfaces, the adhesive layers of the coated samples of Examples 1-20 and 27 are substantially unaffected by the presence of the biological active, and therefore, retained good adhesive strength for use.

Finger Tack Testing for Examples 21-26 and 28

Finger tack tests were performed on the coated samples of Examples 21-26 and 28, pursuant to the above-described method entitled "Finger Tack Test". The coated samples of Example 21, having varying surface coverages of CHG, all exhibited good levels of finger tack. Similarly, the coated samples of Examples 22-26, which used silicone-based PSA's also exhibited good levels-of finger tack. The coated samples exhibited good adhesion to allow them to remain adhered to wound sites, while also exhibiting good removal capabilities. This is-particularly true for the coated samples of Examples 22-26 due to the use of the silicone-based PSA's. As discussed above, silicone-based PSA's offer good skin adhesion characteristics, offer excellent conformability, and provide a gentle release from the skin and wound site. Accordingly, the coated samples of Examples 21-26 are suitable for use as PSA articles, such as PSA wound dressings.

**Claims**

1.   A method of coating an adhesive layer, the method comprising:

   non-contact depositing a fluid solution onto the adhesive layer, the fluid solution comprising a biological active, wherein the fluid solution exhibits a Hildebrand solubility parameter of at least 3.7 MegaPascals$^{1/2}$ greater than a Hildebrand solubility parameter of the adhesive layer; and
   allowing the fluid solution to dry.

2.   The method of claim 1, wherein the biological active is selected from a group consisting of a metal-ion forming compound, a silver-containing compound, a fatty-acid monoester, chlorhexidine gluconate, triclosan, a peroxide, iodine, complexes thereof, derivatives thereof, and combinations thereof.

3.   The method of claim 2, wherein the biological active is fatty acid monoester and the fluid solution further comprises an enhancer selected from a group consisting of chelating agents, organic acids, alcohols, salts thereof, and combinations thereof.

4.   The method of claim 1, wherein the adhesive layer comprises a press-sensitive adhesive.

5. The method of claim 1, wherein the adhesive layer is derived from an adhesive material selected from a group consisting of acrylates, polyurethanes, silicones, natural rubber, polyisoprene, polyisobutylene, butyl rubber, and combinations thereof.

6. The method of claim 1, wherein after the fluid solution substantially dries, the adhesive layer exhibits a peel strength that is at least 70% of a pre-non-contact deposition peel strength exhibited by the adhesive layer, wherein the peel strength is determined pursuant to ASTM D3330.

7. The method of claim 1, wherein the non-contact deposition comprises inkjet printing.

8. The method of claim 1, wherein the non-contact deposition comprises spray atomization deposition.

9. An article comprising:

an adhesive layer; and
a biological active deposited on the adhesive layer by non-contact deposition of a fluid solution comprising the biological active, wherein the fluid solution exhibits a Hildebrand solubility parameter of at least 3.7 MegaPascals$^{1/2}$ greater than a Hildebrand solubility parameter of the adhesive layer.

10. The article of claim 9, wherein after the fluid solution substantially dries, the biological active is concentrated on the adhesive layer at less than 0.78 grams/meter$^2$.


**Patentansprüche**

1. Verfahren zum Beschichten einer Klebstoffschicht, umfassend:

kontaktloses Abscheiden einer Fluidlösung auf der Klebstoffschicht, wobei die Fluidlösung einen biologischen Wirkstoff umfaßt, wobei die Fluidlösung einen Hildebrand-Löslichkeitsparameter aufweist, der um mindestens 3,7 MegaPascal$^{1/2}$ größer ist als ein Hildebrand-Löslichkeitsparameter der Klebstoffschicht; und Trocknenlassen der Fluidlösung.

2. Verfahren nach Anspruch 1, wobei der biologische Wirkstoff aus einer Gruppe bestehend aus einer Metallionen bildenden Verbindung, einer silberhaltigen Verbindung, einem Fettsäuremonoester, Chlorhexidingluconat, Triclosan, einem Peroxid, Iod, Komplexen davon, Derivaten davon und Kombinationen davon ausgewählt wird.

3. Verfahren nach Anspruch 2, wobei es sich bei dem biologischen Wirkstoff um einen Fettsäuremonoester handelt und die Fluidlösung ferner einen Enhancer, ausgewählt aus einer Gruppe bestehend aus Chelatbildnern, organischen Säuren, Alkoholen, Salzen davon und Kombinationen davon, umfaßt.

4. Verfahren nach Anspruch 1, wobei die Klebstoffschicht einen Haftklebstoff umfaßt.

5. Verfahren nach Anspruch 1, wobei sich die Klebstoffschicht von einem Klebstoffmaterial, ausgewählt aus einer Gruppe bestehend aus Acrylaten, Polyurethanen, Silikonen, Naturkautschuk, Polyisopren, Polyisobutylen, Butylkautschuk und Kombinationen davon, ableitet.

6. Verfahren nach Anspruch 1, wobei die Klebstoffschicht nach weitgehendem Trocknen der Fluidlösung eine Schälfestigkeit aufweist, die mindestens 70% einer Schälfestigkeit der Klebstoffschicht vor der kontaktlosen Abscheidung beträgt, wobei die Schälfestigkeit gemäß ASTM D3330 bestimmt wird.

7. Verfahren nach Anspruch 1, wobei die kontaktlose Abscheidung Tintenstrahldruck umfaßt.

8. Verfahren nach Anspruch 1, wobei die kontaktlose Abscheidung Sprühzerstäubungsabscheidung umfaßt-.

9. Artikel mit:

einer Klebstoffschicht und
einem biologischen Wirkstoff, abgeschieden durch kontaktlose Abscheidung einer Fluidlösung des biologischen Wirkstoffs auf der Klebstoffschicht, wobei die Fluidlösung einen Hildebrand-Löslichkeitsparameter aufweist, der um mindestens 3,7 MegaPascal$^{1/2}$ größer ist als ein Hildebrand-. Löslichkeitsparameter der Klebstoffschicht.

**10.** Artikel nach Anspruch 9, wobei der biologische Wirkstoff nach weitgehendem Trocknen der Fluidlösung auf der Klebstoffschicht in einer Konzentration von weniger als 0,78 Gramm/Meter$^2$ vorliegt.

**Revendications**

**1.** Procédé de revêtement d'une couche adhésive, le procédé comprenant :

le dépôt sans contact d'une solution fluide sur la couche adhésive, la solution fluide comprenant un actif biologique, dans lequel la solution fluide présente un paramètre de solubilité de Hildebrand supérieur d'au moins 3,7 mégapascals$^{1/2}$ à un paramètre de solubilité de Hildebrand de la couche adhésive ; et le fait de laisser sécher la solution fluide.

**2.** Procédé selon la revendication 1, dans lequel l'actif biologique est sélectionné dans un groupe constitué d'un composé formant un ion métallique, d'un composé contenant de l'argent, d'un monoester d'acide gras, du gluconate de chlorhexidine, du triclosan, d'un peroxyde, de l'iode, de complexes de ceux-ci, de dérivés de ceux-ci, et de combinaisons de ceux-ci.

**3.** Procédé selon la revendication 2, dans lequel l'actif biologique est un monoester d'acide gras et la solution fluide comprend en outre un agent améliorateur sélectionné dans un groupe constitué d'agents chélatants, d'acides organiques, d'alcools, de sels de ceux-ci, et de combinaisons de ceux-ci.

**4.** Procédé selon la revendication 1, dans lequel la couche adhésive comprend un adhésif sensible à la pression.

**5.** Procédé selon la revendication 1, dans lequel la couche adhésive est dérivée d'un matériau adhésif sélectionné dans un groupe constitué d'acrylates, de polyuréthanes, de silicones, de caoutchouc naturel, de polyisoprène, de polyisobutylène, de caoutchouc butyle, et de combinaisons de ceux-ci.

**6.** Procédé selon la revendication 1, dans lequel après un séchage substantiel de la solution fluide, la couche adhésive présente une résistance au décollement qui correspond à au moins 70 % d'une résistance au décollement présentée par la couche adhésive avant le dépôt sans contact, la résistance au décollement étant déterminée selon ASTM D3330.

**7.** Procédé selon la revendication 1, dans lequel le dépôt sans contact comprend l'impression au jet d'encre.

**8.** Procédé selon la revendication 1, dans lequel le dépôt sans contact comprend le dépôt en utilisant une pulvérisation par atomisation.

**9.** Article comprenant :

une couche adhésive ; et un actif biologique déposé sur la couche adhésive par dépôt sans contact d'une solution fluide comprenant l'actif biologique, dans lequel la solution fluide présente un paramètre de solubilité de Hildebrand supérieur d'au moins 3,7 mégapascals$^{1/2}$ à un paramètre de solubilité de Hildebrand de la couche adhésive.

**10.** Article selon la revendication 9, dans lequel après un séchage substantiel de la solution fluide, l'actif biologique est concentré sur la couche adhésive à un niveau inférieur à 0,78 gramme/mètre carré.

**Fig. 1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 20030054025 A
- US 20030175333 A
- US 242065 A, Cantor
- US 6436420 B, Antelman
- WO 0071183 A, Andrews
- WO 0143549 A, Andrews
- US 6407195 B, Sherman

### Non-patent literature cited in the description

- **D.W. Van Krevelen's.** Properties of Polymers. Elsevier Science, 01 November 1997
- **D.H. Kaeble's.** Computer-Aided Design of Polymer and Composites. Marcel Dekker, Inc, 1985
- **Barton, A. F. M.** Handbook of Solubility and Other Cohesion Parameters. CRC Press, 1991
- **Barton, A, F. M.** Handbook of Polymer-Liquid Interaction Parameters and Solubility Parameters. CRC Press, 1990
- Polymer Handbook. John Wiley, 1989, 519-557
- Applied Polymer Science. ACS Publication 1975
- **Ed. I. Skeist ; V. N. Reinhold.** Handbook of Adhesives. 1990